(19) Europäisches Patentamt
European Patent Office
Office européen des brevets

(11) EP 1 724 588 B1

(12) EUROPEAN PATENT SPECIFICATION

(45) Date of publication and mention
of the grant of the patent:
03.05.2017 Bulletin 2017/18

(51) Int Cl.:
C12Q 1/68 (2006.01)    G01N 33/68 (2006.01)

(21) Application number: 06014441.7

(22) Date of filing: 12.03.2002

(54) **Methods for diagnosing Alzheimer's disease**

Verfahren zum Nachweis von Morbus Alzheimer

Méthodes de diagnostic de la maladie d'Alzheimer

(84) Designated Contracting States:
AT BE CH CY DE DK ES FI FR GB GR IE IT LI LU
MC NL PT SE TR

(30) Priority: 12.03.2001 GB 0106051

(43) Date of publication of application:
22.11.2006 Bulletin 2006/47

(62) Document number(s) of the earlier application(s) in
accordance with Art. 76 EPC:
02704967.5 / 1 370 870

(73) Proprietor: Oxford University Innovation Limited
Botley
Oxford OX2 0JB (GB)

(72) Inventor: Nagy, Zsuzsanna
Edgbaston,
Birmingham B17 8EB (GB)

(74) Representative: Boult Wade Tennant
Verulam Gardens
70 Gray's Inn Road
London WC1X 8BT (GB)

(56) References cited:
WO-A-00/79000      WO-A-97/38133
WO-A-98/27227      WO-A-99/11824
US-A- 5 773 219

• JANICKI S.M. ET AL.: "Presenilin overexpression
arrests cells in the G1 phase of the cell cycle:
Arrest potentiated by the Alzheimer's disease
PS2(N141I) mutant." AM. J. PATHOL., vol. 155, no.
1, July 1999 (1999-07), pages 135-144,
XP002227836 ISSN: 0002-9440
• MCSHEA A. ET AL.: "Abnormal expression of the
cell cycle regulators p16 and CDK4 in Alzheimer's
disease." AM. J. PATHOL., vol. 150, no. 6, June
1997 (1997-06), pages 1933-1939, XP009012038
ISSN: 0002-9440
• ARENDT T. ET AL.: "Neuronal expression of
cycline dependent kinase inhibitors of the INK4
family in Alzheimer's disease." J. NEURAL
TRANSM., vol. 105, 1998, pages 949-960,
XP002431095
• BUSSER J. ET AL.: "Ectopic cell cycle proteins
predict the sites of neuronal cell death in
Alzheimer's disease brain." J. NEUROSCIENCE,
vol. 18, no. 8, 15 April 1998 (1998-04-15), pages
2801-2807, XP002431096
• NAGY Z.: "Cell cycle regulatory failure in
neurones: Causes and consequences"
NEUROBIOL. OF AGING, vol. 21, no. 6, November
2000 (2000-11), pages 761-769, XP002291838
ISSN: 0197-4580
• CULMSEE C. ET AL.: "Hippocampal neurons of
mice deficient in DNA-dependent protein kinase
exhibit increased vulnerability to DNA damage,
oxidative stress and excitotoxicity." MOL. BRAIN
RES., vol. 87, no. 2, 5 March 2001 (2001-03-05),
pages 257-262, XP002443583
• LOVELL M.A. ET AL.: "Decreased base excision
repair and increased helicase activity in
Alzheimer's disease brain." BRAIN RES., vol. 855,
no. 1, 7 February 2000 (2000-02-07), pages
116-123, XP002443584

**(Cont. next page)**

EP 1 724 588 B1

- LOVE S. ET AL.: "Increased poly(ADP-ribosyl)ation of nuclear proteins in Alzheimer's disease." BRAIN, vol. 122 ( Pt 2), February 1999 (1999-02), pages 247-253, XP002233086

**Description**

Field of the invention

The present invention relates diagnostic screens for Alzheimer's disease, and in particular to diagnostic tests based on screening for the presence of cellular changes that occur early in the pathology of Alzheimer's disease.

Background to the invention

As life expectancy increases Alzheimer's disease (AD) is becoming a major health problem in the western world. There has been intensive research aimed at identifying a reliable cure or preventive measures for the disease, so far without success.

One of the biggest problems in the design and testing of any therapeutic agent is the lack of reliable clinical diagnostic criteria that could identify AD sufferers early enough for any meaningful intervention. However, the currently available clinical diagnostic tools do not allow an accurate and reliable diagnosis of Alzheimer's disease in other than severely demented patients. Furthermore they do not allow the identification of subjects with pre-clinical Alzheimer's disease who could benefit from preventive intervention.

The most often used clinical diagnostic criteria are the NINCDS/ADRDA criteria (McKhann, G. et al., (1984) Neurology 34: 939-944), originally designed for research purposes. These criteria are highly sensitive but have a low specificity. This is due to the fact that the positive predictive value of a diagnosis of "probable" or "possible" Alzheimer's disease is very high, but the negative predictive value is very low (13). In other terms, if a patient fulfils the requirements of the NINCDS/ADRDA criteria for Alzheimer's disease it is highly likely that the patient indeed has got Alzheimer's disease. However, a proportion of the patients who do not fulfil these criteria (e.g. are regarded as controls) are found to have Alzheimer's disease at post mortem examination (13).

As a consequence of their low specificity, the NINCDS/ADRDA criteria are not ideal for clinical diagnostic purposes. Additionally they are not suitable as diagnostic criteria for clinical trials looking at preventive or curative therapies that may have their best chance of being effective if used before significant dementia has developed. Thus there remains a need for a reliable diagnostic test for Alzheimer's disease, and in particular for a test which may be used in the early detection of subjects with pre-clinical Alzheimer's disease who could benefit from preventive intervention.

In recent years it is becoming more widely accepted that the pathogenic basis of Alzheimer's disease is the aberrant re-entry of different neuronal populations into the cell division cycle (14). In healthy elderly individuals rapid cell cycle arrest and re-differentiation may follow this cell cycle re-entry. In contrast, in individuals with Alzheimer's disease the regulatory mechanisms appear to fail and the neurons progress into the late stages of the cell cycle leading to the accumulation of AD-related pathology and/or neuronal death (14).

Studies by the present inventors and others indicate that the cell cycle regulatory failure in Alzheimer's disease occurs at the G1/S transition checkpoint (3).

Further studies demonstrate how failure in cell cycle regulation due to alteration of cell cycle regulatory proteins leads to the development of Alzheimer's disease (20). It was also shown that regulators of the cell cycle, cyclin-dependent kinase-4 and its inhibitor cyclin-dependent kinase inhibitor p16 is increased in the brains of cases of Alzheimer's disease (21). Moreover, methods for diagnosing rare early onset familial forms of Alzheimer's disease (showing Mendelian inheritance pattern) by determining variants of the presenilin-1 or presenilin-2 genes are known (22,23).

Previous studies on fibroblasts and lymphocytes from Alzheimer's disease patients indicate that the regulation of the cell division cycle might be disrupted in cells other than neurons in this condition (8, 9, 17). It is also known that Alzheimer's disease patients are more prone to some forms of cancer (4) and that Down's syndrome patients, who develop AD in early adult life, are more prone to leukaemia than the general population (7, 10).

It is plausible therefore to hypothesise that the cell cycle regulatory failure in neurons, even in early (pre-clinical) stages of AD, might be reflected by similar cell cycle regulatory malfunction in lymphocytes.

The present inventors have now shown that the *in vitro* responsiveness of lymphocytes to G1 inhibitor treatment is significantly less effective in Alzheimer's disease patients than in control subjects. Additionally, in subjects showing clinical signs of incipient Alzheimer's disease the lymphocyte-response is similar to that seen in Alzheimer's disease patients. These findings represent direct evidence to support the hypothesis that the failure of the G1/S transition control is not restricted to neurons in Alzheimer's disease patients, but also occurs in peripheral cells, such as lymphocytes.

The observation that the regulatory defect at the G1/S transition also occurs in peripheral cells provides the basis for new clinical tests, useful in the diagnosis of Alzheimer's disease, that rely on eliciting the activation of the G1/S transition checkpoint in non-neuronal cells, such as lymphocyte cultures. Since cell cycle regulatory failure in neurons appears to be a very early event in the pathogenesis of Alzheimer's disease, it is anticipated that such tests will be of use for the identification of subjects in the pre-clinical stages of Alzheimer's disease who do not fulfil the requirements of the NINCDS/ADRDA criteria for dementia, but would benefit from early intervention with preventive measures for Alzheimer's disease.

Description of the invention

In accordance with a first aspect of the invention there is provided a method for diagnosis of Alzheimer's disease in a human subject which comprises screening for the presence of a cell cycle regulatory defect at the G1/S phase transition in non-neuronal cells of the human subject.

The method of the invention is most preferably carried out *in vitro* on non-neuronal cells isolated from the human subject to be tested. The non-neuronal cells may be any non-neuronal cell type which exhibits the same cell cycle regulatory defect at the G1/S phase transition as is present in the neurons in Alzheimer's disease. In the most preferred embodiment the method is carried out on lymphocytes isolated from the subject and cultured *in vitro.* There are obvious practical advantages in being able to test for the presence of the cell cycle regulatory defect in a non-neuronal cell type. The use of lymphocytes is particularly convenient, since they are easily isolated from a blood sample and may be cultured *in vitro.* Another preferred option is the use of fibroblasts, particularly skin fibroblasts which may be conveniently obtained from a skin biopsy.

When the method of the invention is used diagnostically, the presence of a defect in cell cycle regulation at the G1/S phase transition in a non-neuronal cell type is taken as an indication that the subject has Alzheimer's disease. Typically, a reduction in the effectiveness of the checkpoint control at the G1/S transition is taken as an indication that the subject has Alzheimer's disease.

The availability of a reliable test for a defect underlying the pathology of Alzheimer's disease will significantly improve the ability to diagnose the condition, and in particular will enable early diagnosis. The currently available operational diagnostic criteria for Alzheimer's disease only allow diagnosis of possible or probable AD very late, when dementia is already present. A definite diagnosis of Alzheimer's disease can only be made after post mortem examination. It is apparent from the work of the present inventors that a defect in cell cycle control is detectable in peripheral (non-neuronal) cells, such as lymphocytes, well before the clinical signs of fully developed dementia appear. Hence, the method of the invention provides a tool for early diagnosis of Alzheimer's disease, especially detection of individuals who are in pre-clinical stages of the disease, and for identification of individuals who have not yet developed Alzheimer's disease as such but are "at risk" of doing so because of the presence of the cell cycle regulatory defect. This opens up the possibility of early intervention with preventive measures, including, inter *alia*, changes in life style and vitamin regimes and HRT for post menopausal women.

The availability of diagnostic tools capable of detecting early changes in individuals who have not yet developed Alzheimer's disease as such will also facilitate the development and testing of therapies aimed at stopping the progression of the disease at a point before the development of significant brain pathology. The diagnostic tests may also be applied in the development of animal models of early Alzheimer's disease, for example in the identification of a mouse model which exhibits an analogous defect in cell cycle regulation to that present in Alzheimer's disease.

The ability to identify individuals having a cell cycle regulatory defect at the G1/S transition may be applied to the selection of individuals to be included in clinical trials. Clinical trials are more likely to produce meaningful results if the individuals included in the trial are selected to be those most likely to benefit from the treatment under test.

The identification of G1/S regulatory defect in lymphocytes taken from a patient suffering from incipient or full blown Alzheimer's disease may indicate the presence of immune problems in the patient, or a likelihood that the patient will develop immune problems as the disease progresses. The availability of such information will assist the clinician in deciding whether to intervene with therapy aimed at alleviating/preventing immune problems complicating the Alzheimer's disease.

The method of the invention is particularly preferred for diagnosis of sporadic Alzheimer's Disease. However, it would also be useful in the diagnosis of forms of Familial Alzheimer's Disease which exhibit an equivalent cell cycle regulatory defect. Generally this will not include Familial Alzheimer's Disease associated with presenilin-1 or presenilin-2 mutations.

There are several ways in which to screen for the presence of a cell cycle regulatory defect at the G1/S phase transition in non-neuronal cells in accordance with the invention. In one embodiment screening for the presence of the cell cycle regulatory defect may be accomplished by first inducing the cells to divide, then eliciting cell cycle arrest by addition of a cell division inhibitor substance and testing the responsiveness of the cells' G1/S cell cycle regulatory mechanisms to the addition of the cell division inhibitor substance.

Most preferably the cell division inhibitor substance will be a specific G1 inhibitor, for example rapamycin. Cell division may be induced by the addition of a mitogenic stimulus, for example one or more growth factors. If the test is carried out using lymphocytes then phytohaemaglutinin may be used to induce cell division.

In a further, related embodiment treatment with the cell division inhibitor substance may be replaced by treatment with a stimulus which elicits cell cycle arrest at G1, for example an environmental stimulus. Screening for the presence of the G1/S regulatory defect is therefore accomplished by: inducing the cells to divide, exposing the cells to a stimulus which induces cell cycle arrest at G1 and testing the responsiveness of the G1/S cell cycle regulatory mechanisms of the cells to the addition of the stimulus which elicits cell cycle arrest.

Suitable stimuli of cell cycle arrest include, inter *alia*, ionising radiation, hypoxia, UV radiation, etc. In a preferred embodiment, cell cycle arrest may be induced by treatment of the cells with $H_2O_2$ to produce oxidative stress. As above, cell division may be induced by the addition of a mitogenic stimulus, for example one or more growth factors. Phytohaemaglutinin may be used to induce cell division in cultured lymphocytes.

The rationale behind both of these methods is to first stimulate the cells to divide, then attempt to arrest the cell cycle at the G1 stage using either a cell division inhibitor or other stimulus eliciting cell cycle arrest and then evaluate the effect of such treatment on the cell cycle regulatory system. The effect on cell cycle regulation may be evaluated by a variety of different means, as discussed below. The treatment with a cell division inhibitor or other stimulus that induces cell cycle arrest may be referred to herein as "cell cycle inhibitory treatment" or "inhibitory treatment". If a cell cycle regulatory defect at the G1/S transition is present then this will affect the responsiveness of the cells to attempted cell cycle arrest. In general, the presence of a cell cycle regulatory defect at G1/S results in a reduced responsiveness to treatment with a cell division inhibitor or other stimulus that induces cell cycle arrest at G1, i.e. the inhibitory treatment is less effective in arresting the cell cycle at the G1/S checkpoint in cells with such a defect.

Various approaches may be implemented before and after the addition of the mitogenic stimulus and before and after the attempted arrest of the cell cycle to test the responsiveness of the cells to cell cycle inhibitory treatment. A non-exhaustive list of preferred approaches which may be used in accordance with the invention is given below, other suitable approaches will be known to persons skilled in the art:

(1) Proliferation assay performed in order to assess whether cell cycle arrest has occurred and to what extent as a result of inhibitory treatment. The proliferation assay may be carried out according to any of the standard protocols known in the art. A particularly suitable example is the MTT survival assay (commercially available from Chemicon International Ltd, see Mosmann, T. In J. Immunol. Methods, 1983, vol: 65, 55-63).

In a typical screen proliferation assays are performed on both cells treated with a cell division inhibitor or other stimulus inducing cell cycle arrest and untreated control cells from the same subject. Since the inhibitory treatment will be effective only in the presence of an intact G1/S regulatory system, the difference in degree of proliferation between the treated and untreated cells will be significantly smaller in Alzheimer's disease patients than in age matched control individuals. In general, little or no change in the proliferative activity of cells from the subject in the presence of inhibitory treatment indicates a reduced responsiveness to cell cycle inhibition in the G1 phase, and hence the presence of a regulatory defect at the G1/S transition. The presence of such a regulatory defect is in turn taken as an indication that the subject has Alzheimer's disease.

(2) Calculating the relative lengthening of the G1 phase of the cell cycle in cells from the subject as a result of exposure to a cell division inhibitor or stimulus that induces cell cycle arrest. The relative lengthening of the G1 phase as a result of exposure to the cell division inhibitor or stimulus that induces cell cycle arrest is calculated using the formula RL=100f-100 (expressed as a percent). "f" is the ratio of the time in G1 for cells (non-neuronal cells from the subject under test) exposed to inhibitory treatment with the cell division inhibitor or stimulus that induces cell cycle arrest ($TG1_{tr}$) versus the time in G1 for untreated control cells (i.e. also non-neuronal cells from the subject under test) not exposed to inhibitory treatment ($TG1_c$). f may be calculated according to the following relation :

$$f = TG1_{tr}/TG1_c = [\ln 2 - \ln(2 - G1_{tr})][\ln(2 - G1_c)]/[\ln(2 - G1_{tr})][\ln 2 - \ln(2 - G1_c)] \quad (5)$$

Various techniques may be employed to obtain the values of $TG1_{tr}$ and $TG1_c$. In a preferred embodiment $TG1_{tr}$ and $TG1_c$ may be obtained by determining the proportion of cells in the various phases of the cell cycle for both treated cells (non-neuronal cells from the test subject treated with the cell division inhibitor substance or stimulus that induces cell cycle arrest) and untreated control cells (non-neuronal cells from the same subject not exposed to the cell division inhibitor substance or stimulus that induces cell cycle arrest). The proportion of cells in the various phases of the cell cycle may be readily determined by incorporation of a labelled nucleotide analogue, preferably bromodeoxyuridine (BrdU), followed by fluorescence activated cell sorting (FACS analysis), or equivalent, as described in the accompanying examples.

The presence of a cell cycle regulatory defect at the G1/S phase transition is indicated by a reduced relative lengthening of the G1 phase in the presence of the cell division inhibitor substance or stimulus in cells from the test subject, as compared to control cells not having a cell cycle regulatory defect at the G1/S phase transition (see under (1) for further definition of suitable control cells). The control cells not having a cell cycle regulatory defect at the G1/S phase transition are not to be confused with the "untreated control" cells used for calculation of RL, which are cells from the test subject which have not been exposed to inhibitory treatment.

(3) Assessment of cell cycle regulatory protein or mRNA expression. Expression of cell cycle regulatory proteins may be assessed using standard techniques well known in the art such as, for example, immunoblotting, western blotting, ELISA or related methods. Assessment of expression of corresponding mRNAs encoding the cell cycle regulatory proteins may also be accomplished by means of standard methods such as, for example, hybridisation techniques, "DNA chip" analysis or related methods or amplification-based techniques such as RT-PCR or nucleic acid sequence-based amplification (NASBA). Suitable methods for the detection/quantitation of mRNAs which may be used in accordance to the invention will be well known to those skilled in the art. Certain of these methods, for example RT-PCR, rely on detection/quantitation of a cDNA copy of the relevant mRNA.

The cell cycle regulatory defect present in Alzheimer's disease may result in changes in the pattern of expression of cell cycle regulatory proteins, and their corresponding mRNAs. Screening for changes in expression of particular cell cycle regulatory proteins and/or the corresponding mRNAs may therefore be used diagnostically to identify the presence of a cell cycle regulatory defect at G1/S. In addition, expression of cell cycle regulatory proteins may be used as a marker of progression through the cell cycle. Hence, the responsiveness of cells to inhibitory treatment may be assessed by looking at the expression of one or more cell cycle regulatory proteins, in order to determine the extent to which inhibitory treatment causes cell cycle arrest in cells from the test subject. Suitable cell cycle regulatory proteins include CDKN3, p15ink4B, p16ink4A, p19ink4D, p27kip1, p21cip1, p57kip2 and TP53. The sequences of these proteins, and the genes encoding them, are publicly available. A list of OMIM accession numbers for these proteins is provided in the accompanying Examples. Antibodies useful in the detection of each of these proteins are available commercially.

(4) Assessment of cell viability and cell death by any method known in the art. When a proliferating cell is arrested in the G1/S transition one of two possible "downstream" phenomena may result, either differentiation or programmed cell death. These downstream phenomena may be used as an indication of the presence in a cell population of a regulatory defect at the G1/S transition, since if regulation of the G1/S transition is defective then the downstream effects of cell cycle arrest at G1/S will also be abnormal. A lower degree of cell death or higher degree of cell viability in response to inhibitory treatment in cells from the test subject, as compared to control cells, is taken as an indication that the subject has Alzheimer's disease.

(5) Assessment of cell death related (inducing or preventing) protein or mRNA expression using standard techniques. In this embodiment, expression of cell death related proteins, or the corresponding mRNAs, is used as an indirect assessment of the downstream effects of treatment with a cell division inhibitor or other stimulus inducing cell cycle arrest at the G1/S transition. Suitable cell death related proteins include members of the bcl-2 family of proteins, of which there are many known in the art.

(6) Assessment of the DNA content of the non-neuronal cells, with or without cell cycle analysis. In this embodiment, measurement of the DNA content of cells from the test subject treated with a cell division inhibitor or other stimulus inducing cell cycle arrest provides an indirect indication of the presence of a regulatory defect at the G1/S transition in such cells. The rationale behind this method is the difference in DNA content between cells in the G1 phase and cells in the G2 phase which have passed through the DNA replication stage of the cell cycle. When a population of normal cells (i.e. without a regulatory defect at G1/S) are treated to induce cell cycle arrest in G1 or at G1/S, the majority of the cells will remain in the G1 phase. However, if cells have a regulatory defect at G1/S, a proportion of the cells will pass through the G1/S checkpoint and undergo DNA replication. Thus an increased DNA content in cells from a test subject, as compared to control cells not having a regulatory defect at G1/S, following treatment to induce cell cycle arrest at G1 is taken as an indication of the presence of a regulatory defect at G1/S. The presence of such a regulatory defect is in turn taken as an indication that the subject has Alzheimer's disease.

The above list of techniques suitable for use in testing the responsiveness of non-neuronal cells, particularly cultured lymphocytes, to inhibitory treatment with a cell division inhibitor or stimulus that induces cell cycle arrest is intended to be illustrative of rather than limiting to the invention.

In a second aspect the invention provides a method for use in diagnosis of Alzheimer's disease in a human subject which comprises screening for the presence in the genome of said subject of at least one mutation or allelic variant in a cell cycle regulatory gene, wherein the presence of a mutation or allelic variant in a cell cycle regulatory gene is taken as an indication of Alzheimer's disease.

Most preferably, the method of the invention will involve screening for the presence of at least one mutation or allelic variant in a cell cycle regulatory gene selected from the group consisting of CDKN3, p15ink4B, p16ink4A, p19ink4D, p27kip1, p21cip1, p57kip2 and TP53.

The method of the invention is not limited to screening for the presence of any specific mutation or genetic variant, although screening for the presence of specific mutations/variants shown to be associated with Alzheimer's disease is contemplated. The invention encompasses scanning the cell cycle regulatory gene, or a sub-region thereof, for the presence of mutations or genetic variants, including previously unknown mutations/variants. For the avoidance of doubt, the term "gene" includes the regulatory regions, in particular the promoter region. The presence of one or more mutations or genetic variants in a cell cycle regulatory gene, particularly mutations/variants which result in a change in the amino acid sequence of the protein encoded by the gene or which alter the function of the encoded protein or which alter the level of expression of the protein, is taken as an indication that the individual has Alzheimer's disease, on the basis that the presence of such a mutation or variant is indicative of a cell cycle regulatory defect.

There are many techniques known in the art for detection of genetic variation which may be used, in accordance with the invention, to scan a cell cycle regulatory gene of a given human subject for the presence of mutations/allelic variants. Suitable techniques include, for example, single strand confirmation polymorphism analysis (SSCP), PCR-SSCP heteroduplex analysis (HA), denaturing gradient gel electrophoresis (DGGE), DNA sequencing, RNase cleavage, chemical cleavage of mismatch (CCM) etc (see review by Schafer and Hawkins, Nature Biotechnology, Vol: 16, pp33-39, 1998).

The accompanying Example 2 describes the use of PCR-SSCP to screen for polymorphic variants in the p21cip and p57 genes and thereby identify a polymorphic variant in exon 2 of the p21cip gene and two polymorphic variants in exon 2 of the p57 gene. The same technical approach may be employed to screen for polymorphic variants in other genes with appropriate modification, i.e. the selection of PCR primers specific for the gene of interest.

Scanning for the presence of mutations/allelic variants is carried out on a sample of genomic DNA isolated from the human subject. Genomic DNA may be conveniently isolated from a whole blood sample using standard techniques well known in the art. Advantageously, the process of scanning for the presence of mutations/allelic variants may be carried out on genomic DNA prepared from cultured lymphocytes from the subject. The same culture of lymphocytes may also be tested "functionally" for the presence of a cell cycle regulatory defect at the G1/S phase transition, for example by testing responsiveness to inhibitory treatment using a method according to the first aspect of the invention.

Associations between a given polymorphic variant and susceptibility to Alzheimer's disease may be confirmed by carrying out genetic association studies, for example family-based or case-control association studies. The disease association of particular polymorphic variants may also be determined by evaluated the relationship between genotype and expression of markers of cell cycle progression in the brain. In the accompanying examples, the relationship between genotype and expression of cyclin B (a marker of progression though the cycle to the G2 stage) in neuronal nuclei was evaluated. Other markers of cell cycle progression could have been used with equivalent effect.

In a specific embodiment, the method may involve genotyping for one of the polymorphic variants in the p21 and p57 genes described in the accompanying examples. These variants, denoted p21E2 alleles A and B, p57E2A alleles A and B, and P57E2B alleles A and B, may be genotyped on the basis of PCR-SSCP analysis using the following primer sets under the conditions specified in the accompanying examples:

p21E2  A/B:  5'-CGGGATCCGGCGCCATGTCAGAACCGGC-3'  (SEQ  ID  NO: 1)  and  5'-CCAGACAGGTCAGCCCTTGG-3' (SEQ ID NO: 2)

P57E2A A/B: 5'-GGC CAT GTC CGA CGC GTC-3' (SEQ ID NO: 3) and 5'-AGG CGG CAG CGC CCC ACC TG-3' (SEQ ID NO: 4)

p57E2B A/B: 5'-ATT ACG ACT TCC AGC AGG ACA TG-3' (SEQ ID NO: 5) and 5'-CTG GAG CCA GGA CCG GGA CTG-3' (SEQ ID NO: 6)

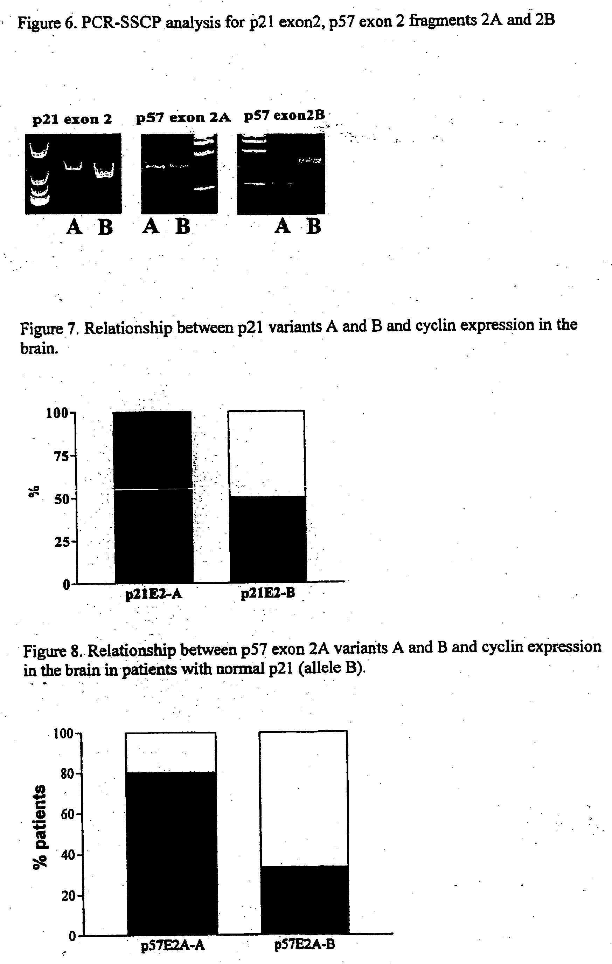

In each case, the A and B alleles may be identified on the basis of differential electrophoretic mobility of the resultant PCR products under the conditions defined in Example 2, with reference to Figure 6. It is, however, not intended to limit the invention to the use of PCR-SSCP and other methods of genotyping the same variants may be used.

The p21E2 A allele and the p57E2A A allele are both associated with increased progression through the G1/S checkpoint, and therefore with susceptibility to Alzheimer's disease.

Screens based on detection of the presence of mutations or allelic variants in genes encoding cell cycle regulatory genes may involve genotyping for two or more polymorphic variants, or may involve scanning one or more cell cycle regulatory genes for the presence of genetic variation. Any genetic variation which has an adverse effect on the function of a cell cycle regulatory gene may potentially result in by-pass of the G1/S transition check point, and consequential AD pathology. Accumulation of genetic variation within a single regulatory gene, or across multiple genes, may have an additive effect.

Screens based on detection of the presence of mutations or allelic variants in genes encoding cell cycle regulatory genes may be used in the diagnosis of Alzheimer's disease, possibly in conjunction with other diagnostic tests, such as screening for the presence of a cell cycle regulatory defect. They may also be used in order to identify individuals having a pre-disposition to Alzheimer's disease because of the presence of the mutation(s) or allelic variant(s) in a cell cycle regulatory gene.

The approach of screening for mutations or allelic variants in a cell cycle regulatory gene may also be used in order to determine any genetic basis for Alzheimer's disease in a patient previously diagnosed with Alzheimer's disease.

In a still further aspect, the disclosure describes a method of identifying compounds having potential pharmacological activity in the treatment of Alzheimer's disease, which method comprises steps of:

analysing the regulation of the G1/S transition in non-neuronal cells, which cells exhibit a cell cycle regulatory defect at the G1/S phase transition, in the presence and absence of a test compound, wherein a test compound which results in correction of the regulatory defect at the G1/S transition in said cells is identified as having potential pharmacological activity in the treatment of Alzheimer's disease.

The method may be performed using essentially any non-neuronal cells which exhibit an analogous cell cycle regulatory defect at the G1/S phase transition to that observed in the neurons in Alzheimer's disease. Suitable cells may include cultured lymphocytes derived from an individual, or several individuals, having Alzheimer's disease.

"Analysis" of the regulation of the G1/S transition may be performed using any of the methods described in connection with the first aspect of the invention as suitable for screening for the presence of a cell cycle regulatory defect at G1/S. Advantageously, the method used for analysis of the regulation of the G1/S phase transition will be one capable of being performed in multi-well microtiter plates, allowing the compound screen to be carried out in mid-to-high throughput format. The most preferred method suitable for use in a mid-to-high throughput format is the cell proliferation assay.

Cells exhibiting a regulatory defect at the G1/S transition are exposed to test compounds and the effect of the test compound on regulation of the G1/S transition is assessed with reference to suitable controls, e.g. cells not exposed to any test compound. In a typical screen, the test compound will be tested at a range of different concentrations.

There is no limitation on the types of candidate compounds to be tested in the screening methods of the invention. Test compounds may include compounds having a known pharmacological or biochemical activity, compounds having no such identified activity and completely new molecules or libraries of molecules such as might be generated by combinatorial chemistry. Compounds which are DNA, RNA, PNA, polypeptides or proteins are not excluded.

The basic compound screening methodology may also be adapted for use in assessing the efficacy of a form of treatment for Alzheimer's disease, for example to test the effect of a particular pharmacological agent on cell cycle regulation.

In a useful variation, the method may be used specifically to determine whether a pharmacological agent is likely to be of benefit in the treatment of Alzheimer's disease in a particular human individual. In this case the assay is performed using non-neuronal cells from the individual that exhibit a cell cycle regulatory defect at the G1/S phase transition, most preferably cultured lymphocytes. The cells are tested for the presence of the defect in regulation at the G1/S phase transition at the G1/s transition in the presence and absence of the pharmacological agent. Pharmacological agents which result in "correction" of the regulatory defect at the G1/S transition are identified as likely to be of benefit in the treatment of Alzheimer's disease in the individual. By "correction" is meant a significant degree of restoration to normal cell cycle regulation. This may be assessed by reference to control cells, for example cells of the same type taken from an age-matched control individual not having Alzheimer's disease or any evidence of a regulatory defect at the G1/S transition or any genetic defect/allelic variation which might be expected to pre-dispose to Alzheimer's disease.

The invention will be further understood by reference to the following experimental examples, together with the accompanying Figures, in which:

Figure 1 illustrates flow cytometer readouts for cultured lymphocytes from a control subject, preAD subject and AD patient. Results are shown for both rapamycin treated cells and control, untreated cells. M1 indicates that the cells are in the G1 phase of the cell cycle.

Figure 2 illustrates relative (left panel) and age-corrected relative (right panel) lengthening of the G1 phase of the cell cycle under the influence of rapamycin in cultured lymphocytes from preAD, AD, ADM, possAD, DNOS and control subjects.

Figure 3 illustrates the effects of 24 hours rapamycin treatment on cell survival in cultured lymphocytes from preAD, AD, possADM, DNOS and control subjects. Absolute values are shown in the left panel, age-corrected values in the right panel.

Figure 4 illustrates the effects of doxorubicin treatment on cell survival in cultured lymphocytes from preAD, AD, possADM, DNOS and control subjects Absolute values are shown in the left panel, age-corrected values in the right panel.

**Figure 5** illustrates the effects of $H_2O_2$ treatment on cell survival in cultured lymphocytes from preAD, AD, possADM, DNOS and control subjects. Absolute values are shown in the left panel, age-corrected values in the right panel.

**Figure 6** illustrates the result of PCR-SSCP screening for polymorphisms in exon 2 of p21 and exon 2 of p57.

**Figure 7** illustrates the relationship between p21 variants A and B and cyclin B expression in the brain. White bar indicates the percent of patients where cyclin B was NOT expressed in neurones. Black bars indicate the percent of patients where cyclin B was expressed in neuronal nuclei.

**Figure 8** illustrates the relationship between p57 exon 2A variants A and B and cyclin expression in the brain in patients with normal p21 (variant B). White bar indicates the percent of patients where cyclin B was NOT expressed in neurones. Black bars indicate the percent of patients where cyclin B was expressed in neuronal nuclei.

**Figure 9** illustrates the prevalence of somatic mutations in relation to AD progression and cell cycle proteins in the brain. x axis: Braak stages of AD severity; y axis: percent primers that generated differing RAPD patterns from blood when compared to brain.

**Key to Figures:**

Control:healthy individuals with normal cognitive and neuropsychological test results;
PreAD: healthy individuals with neuropsychological test results suggestive of incipient AD;
PossAD: possible Alzheimer's disease as diagnosed by the NINCDS criteria;
AD: probable Alzheimer's disease as diagnosed by the NINCDS criteria;
ADM: possible Alzheimer's disease (NINCDS) and evidence of other type of dementia;
DNOS: patients with dementia who do not meet the requirements of the NINCDS criteria for probable Alzheimer's disease.

Example 1-evidence for the occurrence of a cell cycle regulatory defect in lymphocytes of Alzheimer's disease patients

Materials and methods

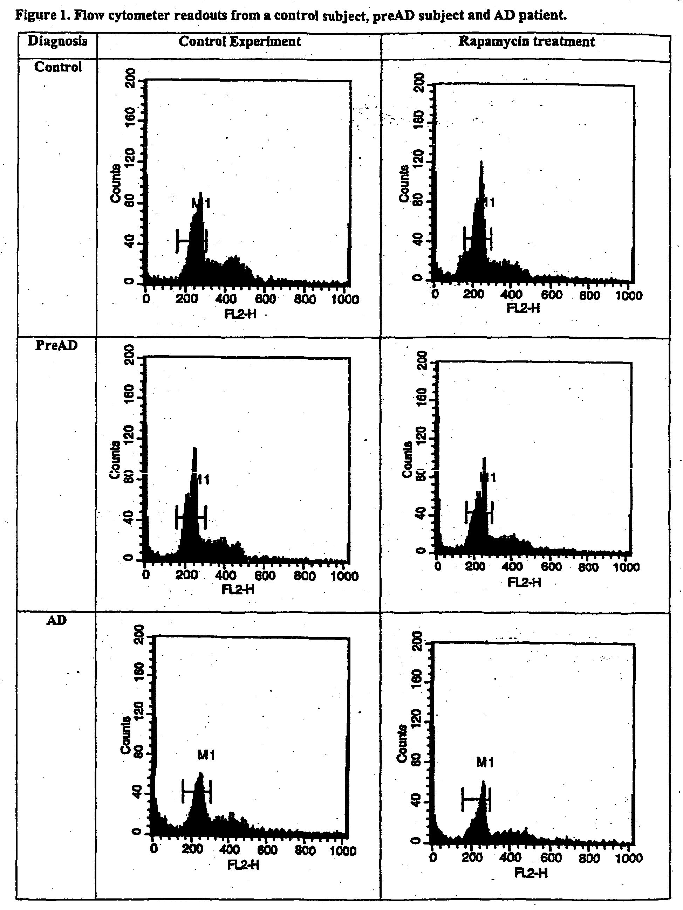

The study included 102 subjects who were full participants of the Oxford Project to Investigate Memory and Ageing (OPTIMA). The yearly routine OPTIMA examination includes a physical examination, cognitive and neuropsychological testing. Drug intake and any intercurrent infections are recorded. Blood was collected in lithium heparin or EDTA vacutainers. Lymphocytes were isolated according to a standard protocol using Ficall (Sigma). In order to standardise the culture methods for all patients the separated lymphocytes were frozen and stored for further analysis.
When the lymphocytes were needed for culture, they were thawed in a 37°C water bath and washed twice in RPMI (any medium or buffer which supports lymphocyte survival may be used to wash the cells with equivalent effect). Cell viability (Trypan Blue exclusion) was typically approximately 80-90%.
A first set of experiments was carried out on 49 subjects (Table 1a), whilst a second set of experiments was carried out on 55 subjects (Table 1b).
In the first set of experiments (49 subjects) two parallel lymphocyte cultures were set up from every individual in RPMI medium supplemented with 10% FCS at a concentration of $1 \times 10^6$ cells per 1ml of culture media. Phytohaemaglutinin (PHA) was added to the cultures at a final concentration of 22 $\mu$g/ml to activate the lymphocytes. Cultures were incubated for 48 hours at 37°C in a humidified atmosphere containing 5% $CO_2$. After 48 hours incubation one culture was treated with 100ng/ml rapamycin, while the other untreated culture was kept as a control. After a further 23 hours incubation BrdU was added in a concentration of 10 $\mu$g/ml to all cultures. After another hour cultures were 'collected' and fixed in 70% ice-cold ethanol. BrdU incorporation was assessed using immunohistochemistry followed by FACS analysis. The proportion of cells in various phases of the cell cycle was determined and data transformation performed to obtain the relative lengthening of the G1 phase (Figure 1).
The calculations (relative lengthening of the G1 phase of the cell cycle in treated cultures relative to control cultures) were based on the assumptions that cells were in the exponential phase of proliferation and that the growth fraction in the cultures (ratio of dividing cells versus quiescent cells) was 1.0 (5). It was also assumed that rapamycin only altered the length of the G1 phase (19). Based on these assumptions the relative lengthening of the G1 phase was calculated using the formula: RL=100f-100 (expressed in percent). The ratio of the G1 time in treated versus control cultures:

$$f=TG1_{tr}/TG1_c=[ln2-ln(2-G1_{tr})][ln(2-G1_c)]/[ln(2-G1_{tr})][ln2-ln(2-G1_c)] \quad (5).$$

In the second set of experiments (53 subjects) after an initial 48 hours incubation (as above) four separate cultures were set up. Control cultures were left without any treatment, one set of cultures was treated with 100ng/ml rapamycin, the third set was treated with 1 $\mu$M doxorubicin while the fourth set was treated with 120 $\mu$M H$_2$O$_2$. Doxorubicin induces DNA damage, leading to arrest at G2/M, rather than G1/S. H$_2$O$_2$ treatment produces oxidative stress, leading to a reversible and temporary cell cycle arrest at G1/S.

After 20 hours of incubation a 4 hour long MTT survival assay (Chemicon International Ltd) was set up. Results were read using a microplate reader (570 filter 630 reference filter). The ratio between cell numbers in treated cultures versus controls was expressed as percent.

All experiments were carried out blind to the clinical diagnosis of the patients.

The results were analysed in relation to the clinical diagnosis provided by the clinicians involved in the OPTIMA project.

Statistical analysis was carried out using the Statgraphics software package. ANOVA tests were performed to examine the effect of the clinical diagnosis on the cell culture parameters. A second set of analyses was also carried out to control for the effect of age on the results. Age correction allows for any age-related variation in the particular parameter under test. The effect of age on a given parameter is determined by looking at the effects of age on the parameter in healthy subjects.

Results

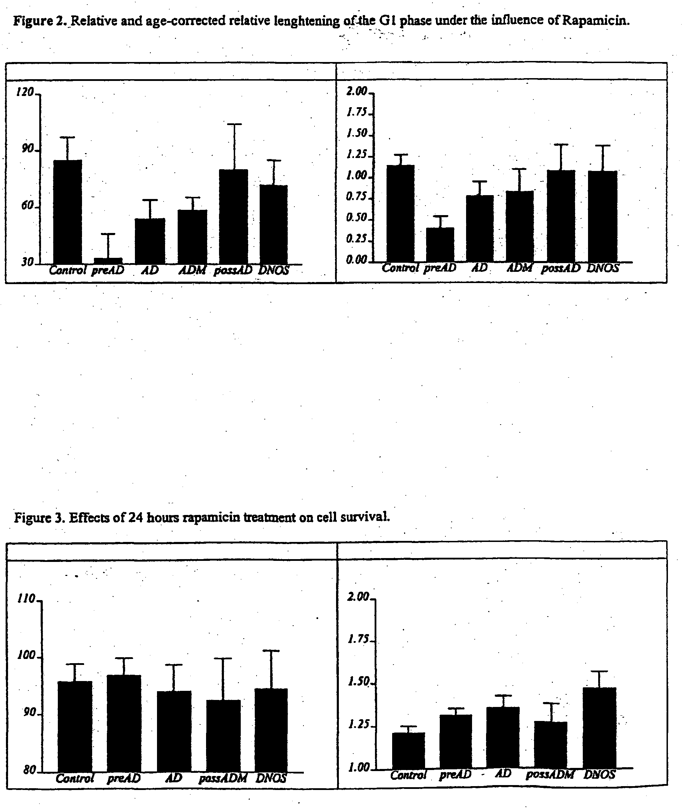

The clinical diagnoses of the patients included in the two sets of experiments are summarised in Tables 1a and 1b. The relative lengthening of the G1 phase under the influence of the specific G1 inhibitor rapamycin was significantly dependent on the clinical diagnosis of our patients (Anova p=0.04, Kruskall-Wallis test p=0.017) (Figure 2). The highest values, indicating more effective G1 inhibition by rapamycin, were found in subjects diagnosed as controls, dementia syndromes other than AD and possible Alzheimer's disease patients. Patients diagnosed as suffering from AD (probable AD by NINCDS) and those with AD and coexisting other pathology (ADM) were found to have a significantly less effective G1 block than control subjects and patients suffering from DNOS or possAD as diagnosed by the NINCDS criteria (Figure 2). The differences between clinical diagnostic categories are similar even when the relative G1 delay was corrected for age (Figure 2).

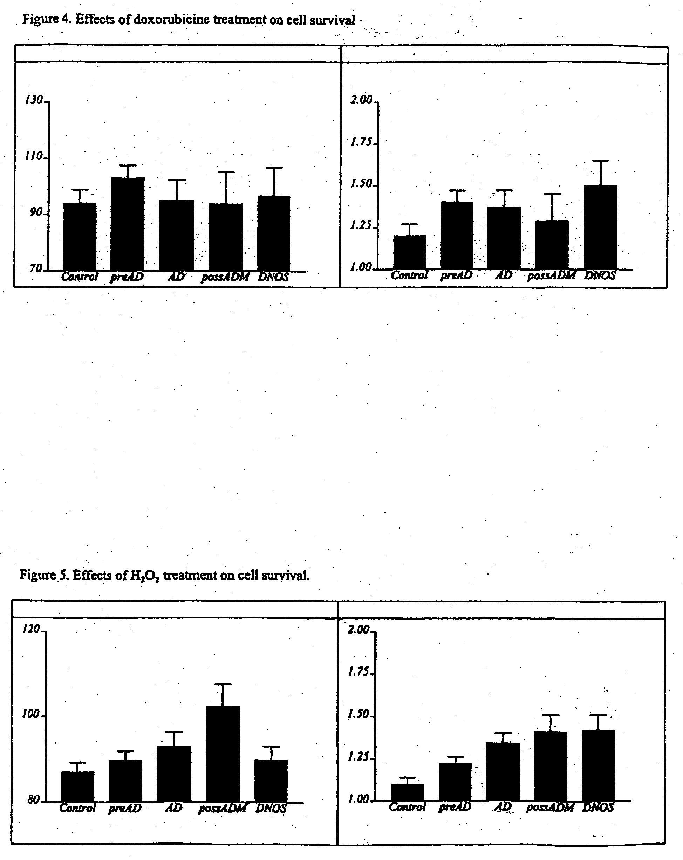

In the second set of experiments the cell numbers after rapamycin treatment showed the expected pattern of higher cell numbers in AD and poss AD cultures, as compared to control cultures (Figure 3). However, the sensitivity of the MTT assay system is relatively low. Doxorubicin treatment did not result in significant differences between patient groups (Figure 3). In contrast to doxorubicin treatment, the reduction of cell numbers by H$_2$O$_2$ treatment was dependent upon the clinical diagnosis. The patients suffering from AD, preAD and possAD had significantly higher cell numbers than control subjects as a result of the H$_2$O$_2$ treatment. DNOS patients showed wide variations and were not different from either of the other patient groups (Figure 4).

**Table 1a-Patients included in this study**

| Clinical diagnosis | No. of patients |
|---|---|
| Control | 14 |
| PreAD | 13 |
| PossAD | 3 |
| AD | 9 |
| ADM | 7 |
| DNOS | 3 |

**Table 1b**

| Clinical diagnosis | No. of patients |
|---|---|
| Control | 16 |
| PreAD | 18 |
| ADM | 3 |
| AD | 11 |
| DNOS | 5 |

KEY:

Control:healthy individuals with normal cognitive and neuropsychological test results;
PreAD: healthy individuals with neuropsychological test results suggestive of incipient AD;
PossAD: possible Alzheimer's disease as diagnosed by the NINCDS criteria;
AD: probable Alzheimer's disease as diagnosed by the NINCDS criteria;
ADM: possible Alzheimer's disease (NINCDS) and evidence of other type of dementia;
DNOS: patients with dementia who do not meet the requirements of the NINCDS criteria for probable Alzheimer's disease.

Discussion

In this study the effects of a specific G1 inhibitor (rapamycin) (16, 19) on the length of the G1 phase in lymphocytes were analysed using BrdU incorporation assay and FACS analysis. The reduction of cell numbers in the cultures following rapamycin treatment was also assessed.
In a second approach, G1 inhibition was elicited by oxidative stress and the reduction of cell numbers in the culture system measured.

The results of the first set of experiments indicate that G1 inhibitor-induced cell cycle arrest, as indicated by the lengthening of the G1 phase of the cell cycle, is significantly less effective in patients suffering from Alzheimer's disease than in control individuals. It is also apparent that these changes appear early, at the stage when the patients are not yet clinically demented but show signs of specific rapid memory loss, known to be the first explicit sign of dementia.

The rapamycin induced G1 block depends on the expression and activity of the p27kip1 CDKI that inhibits the activity of the CyclinE/cdk2 complex (16, 19). Therefore the relative lengthening of the G1 phase under the influence of rapamycin, will depend upon the expression/activity of this CDKI (19). The fact that this relative lengthening of G1 is significantly lower in Alzheimer's disease patient than in healthy elderly individuals would support our hypothesis that a G1/S checkpoint failure in neurons might be accompanied by similar cell cycle control damage in other cell types in these patients. It is also apparent that the failure in cell cycle control is detectable in peripheral cells well before the signs of fully developed dementia appear. This makes it possible that an assay based on the mitogenic stimulation of peripheral lymphocytes followed by an attempted G1/S transition block could be used for the early detection of patients who may be at risk for developing AD-type brain pathology later.

The differences in cell numbers induced by $H_2O_2$ were significantly different in our patient groups indicating that lymphocytes from control subjects have a more pronounced response to oxidative stress. Treatment with 120 mM $H_2O_2$ has been shown to induce a reversible and temporary cell cycle arrest at the G1/S transition point (6) to allow time for DNA damage-repair. Our results indicate that this mechanism is not fully efficient in Alzheimer's disease patients. This inadequate response to oxidative stress is also present in subjects suffering from preclinical AD. The altered response to oxidative stress may also be a reflection of more substantial pre-existing DNA damage in the AD patients (11). However, this possibility is excluded by the fact that doxorubicin-induced DNA damage, inducing a G2/M arrest rather than a G1 arrest, is not different in our patient groups. The results of this study are therefore interpreted as indicating a specific failure in the regulation of the G1/S transition point.

In summary, the results of this study indicate that the response of activated lymphocytes to G1 inhibition is significantly altered in Alzheimer's disease sufferers. In addition these alterations appear early before the onset of a fully developed dementia syndrome identifying subjects who are likely to develop Alzheimer' disease later. The results indicate that a diagnostic test relying on the detection of the integrity of the G1/S transition checkpoint may allow the identification of subjects who are at risk from developing AD later. The advantage of this diagnostic procedure would lie in its ability to identify this group, opening the possibility of preventive intervention for these people.

The protocols described under Example 1 for separation and culture of lymphocytes, induction of cell division, induction of cell cycle arrest by either treatment with a cell division inhibitor or $H_2O_2$-induced hypoxia, BrdU incorporation/FACS analysis and MTT survival assay, or minor adaptations thereof, may all be used diagnostically to test for the presence of a regulatory defect at the G1/S transition.

Example 2-identification of single nucleotide polymorphisms in p21cip and p57

Methods

The exon 2 of p21cip was amplified from genomic DNA (extracted from brain or blood) using 5'-CGGGATCCGGCGCCATGTCAGAACCGGC-3' (SEQ ID NO: 1) and 5'-CCAGACAGGTCAGCCCTTGG-3' (SEQ ID NO: 2) primers. PCR amplification was carried out in a final volume of 50 $\mu$l using 1.25 units of Taq DNA polymerase, 1.5 mM $MgCl_2$, 5% Gelatine, 200 $\mu$M of each dNTP in PCR buffer (75 mM Tris-HCl, pH 8.8, 20 mM $(NH_4)_2SO_4$ and 0.01% Tween). The hot start (95°C for 5') was followed by 30 cycles of 95°C 1 min, 65°C 1 min, 72°C 1 min.

The first segment of exon 2 of p57 (exon 2Ap57) was amplified from genomic DNA (extracted from brain or blood) using 5'-GGC CAT GTC CGA CGC GTC-3' (SEQ ID NO: 3) and 5'-AGG CGG CAG CGC CCC ACC TG-3' (SEQ ID NO: 4) primers.
PCR amplification was carried out in a final volume of 50 $\mu$l using 1.25 units of Taq DNA polymerase, 1.5 mM $MgCl_2$, 10% DMSO, 200 $\mu$M of each dNTP in PCR buffer (75 mM Tris-HCl, pH 8.8, 20 mM $(NH_4)_2SO_4$ and 0.01% Tween). The hot start (95°C for 5 min) was followed by 30 cycles of 95°C 30s, 52°C 30s, 72°C 30s.

The second segment of exon 2 of p57 (exon 2Bp57) was amplified from genomic DNA (extracted from brain or blood) using 5'-ATT ACG ACT TCC AGC AGG ACA TG-3' (SEQ ID NO: 5) and 5'-CTG GAG CCA GGA CCG GGA CTG-3' (SEQ ID NO: 6) primers. PCR amplification was carried out in a final volume of 50 $\mu$l using 1.25 units of Taq DNA polymerase, 1.5 mM $MgCl_2$, 200 $\mu$M of each dNTP in PCR buffer (75 mM Tris-HCl, pH 8.8, 20 mM $(NH_4)_2SO_4$ and 0.01% Tween). The hot start (95°C for 5 min) was followed by 30 cycles of 95°C 30s, 53°C 30s, 72°C 30s.

For initial screening the 4 $\mu$l PCR product was added to 16 $\mu$l of denaturation solution (95% deionised formamide, 10mM NaOH, 0.01% bromophenol blue, and 0.01% xylene cyanol FF) corresponding to 20 $\mu$l for gel loading. The mixture was incubated for 5 min at 95°C and applied to a metaphor agarose gel (2% for exon 2 p21, and 3% for exon 2A p57 and exon2B p57) containing 1:10000 Gelstar. The electrophoresis apparatus was maintained in a standard refrigerator at a constant temperature of 4°C. Electrophoresis was carried out using 1X TBE (45 mM Tris-borate/1 mM EDTA) at 5 V/cm for 2 hours. SSCP patterns appearing on the gel were detected by UV light.

Results

Polymorphisms were found on the exon 2 of the p21 cip and exon 2 of the p57 kip2 genes (Figure 6). The polymorphism detected on the exon 2 of p21cip (variant A) was significantly associated with cell cycle de-regulation in neurones (Figure 7), i.e. the progression of the cell cycle through the G1/S transition into the G2 phase, as indicated by cyclin B positivity. In patients with normal p21cip (variant B) the polymorphism on p57 exon 2A is associated with cyclin B positivity (Figure 8), indicating progression of the cell cycle through the G1/S transition into the G2 phase.

Example 3-RAPD screening for somatic mutations

Methods

Somatic mutations in neurones were screened using genetic fingerprinting methods. DNA was obtained from the brain and the blood of all patients. PCR amplification was carried out on both samples from each patient. 10 short primers were used to randomly amplify polymorphic DNA sequences (primers listed in table 2). PCR amplification was carried out in a final volume of 50 $\mu$l using 1.25 units of Taq DNA polymerase, 1.5 mM $MgCl_2$, 200 $\mu$M of each dNTP in PCR buffer (75 mM Tris-HCl, pH 8.8, 20 mM $(NH_4)_2SO_4$ and 0.01% Tween). The hot start (95°C for 5 min) was followed by 40 cycles of 94°C 30s, AnT 1 min, 72°C 2 min. The annealing temperature (AnT) varied between 33°C and 39°C depending on the primer. The PCR product was applied to a 2% agarose gel containing 1:10000 Gelstar. Electrophoresis was carried out using 1X TBE (45 mM Tris-borate/1 mM EDTA) at 6.5 V/cm for 2 hours. The RAPD sequence patterns were detected by UV light. The differences between the RAPD sequence from blood and brain DNA were compared for each patient and expressed as the % primers that led to different RAPD profiles (RAPD profiles not shown).

Results

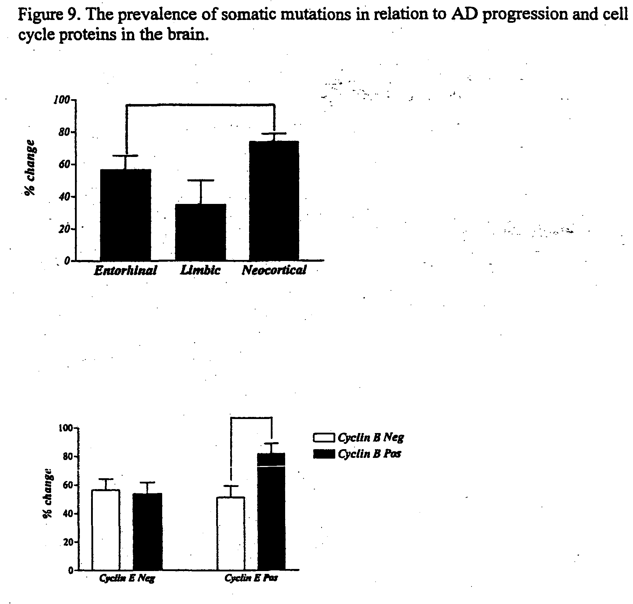

The amount of somatic mutations in the brain in AD patients was observed to be significantly associated with cell cycle deregulation and severity of AD-type pathology (Figure 9). Therefore, it is suggested that DNA-repair insufficiency may lead to the de-regulation of the G1/S transition point finally leading to the development of Alzheimer's disease.

The genotyping findings are in concordance with previous findings in lymphocytes from AD patients that indicate that there is a detectable dysfunction at the G1/S regulation control elicited either by CDKI inducing inhibitors or by oxidative DNA damage.

Table 3-primers used for RAPD analysis

| Sequence | SEQ ID NO: |
|---|---|
|  |  |
| 5'-CCGGCTACGG-3' | 7 |
| 5'-CAGGCCCTTC-3' | 8 |
| 5'-TACGGACACG-3' | 9 |
| 5'-AGCTTCAGGG-3' | 10 |
| 5'-AGGCATTCCC-3' | 11 |
| 5'-GGTCTGAACC-3' | 12 |
| 5'-TAGGCTCACG-3' | 13 |
| 5'-ACGGTACACT-3' | 14 |
| 5'-GTCCTCAACG-3' | 15 |
| 5'-CAATGCGTCT-3' | 16 |

OMIM accession numbers:

| Gene/protein | Accession number |
|---|---|
| CDKN3 | 123832 |
| p15ink4B | 600431 |
| p16ink4A | 600160 |
| p19ink4D | 600927 |
| p27kip1 | 600778 |
| p21cip1 | 116899 |
| p57kip2 | 600856 |
| TP53 | 191170 |
| Gadd45A | 126335 |
| Gadd45B | 604948 |
| Gadd45G | 604949 |
| Gadd153 | 126337 |
| PCNA | 176740 |
| Ku70 | 152690 |
| KU80 | 194364 |
| Ku86 | 604611 |
| NDHII | 603115 |
| BLM | 604610 |
| RECQL | 600537 |
| RECQL4 | 603780 |
| RECQL5 | 603781 |

Cited references

1. Araga S, Kagimoto H, Funamoto K and Takahashi K (1990) Jpn J Med 29: 572-5
2. Arendt T, Holzer M and Gartner U (1998) J Neural Transm 105: 949-60
3. Arendt T, Rodel L, Gartner U and Holzer M (1996) Neuroreport 7: 3047-9
4. Burke WJ, McLaughlin JR, Chung HD, Gillespie KN, Grossberg GT, Luque FA and Zimmerman J (1994) Alzheimer Dis Assoc Disord 8: 22-8
5. Darzynkiewicz Z (1993) In Fantes P and Brooks R (eds) The cell cycle. Oxford University Press, Oxford, pp 43-68
6. Davies KJ (1999) IUBMB Life 48: 41-7
7. Drabkin HA and Erickson P (1995) Prog Clin Biol Res 393: 169-76
8. Eckert A, Hartmann H, Forstl H and Muller WE (1994) Life Sci 55: 2019-29
9. Fischman HK, Reisberg B, Albu P, Ferris SH and Rainer JD (1984) Biol Psychiatry 19: 319-27
10. Fong CT and Brodeur GM (1987) Cancer Genet Cytogenet 28: 55-76
11. Mecocci P, Polidori MC, Ingegni T, Cherubini A, Chionne F, Cecchetti R and Senin U (1998) Neurology 51: 1014-1017
12. Melaragno MI, Smith MdA, Kormann Bortolotto MH and Toniolo Neto JT (1991) Gerontology 37: 293-8
13. Nagy Z, Esiri MM, Hindley NJ, Joachim C, Morris JH, King EM-F, McDonald B, Litchfield S, Barnetson L, Jobst KA and Smith AD (1998) Dementia 9: 219-226
14. Nagy Z, Esiri MM and Smith AD (1998) Neuroscience 84: 731-739
15. Payao SL, Smith MD and Bertolucci PH (1998) Gerontology 44: 267-71
16. Sherr CJ (1994) Stem Cells 12: 47-55; discussion 55-7
17. Tatebayashi Y, Takeda M, Kashiwagi Y, Okochi M, Kurumadani T, Sekiyama A, Kanayama G, Hariguchi S and Nishimura T (1995) Dementia 6: 9-16
18. Trieb K, Ransmayr G, Sgonc R, Lassmann H and Grubeck Loebenstein B (1996) Neurobiol Aging 17: 541-7
19. Wagner EF, Hleb M, Hanna N and Sharma S (1998) J Immunol 161: 1123-31
20. Nagy Z (2000) Neurobiol Aging 21: 761-9
21. McShea A, Harris PLR, Webster KR, Wahl AF, Smith MA (1997) Am J Pathol 150: 1933-9
22. WO 00/79000
23. WO 97/38133

## Claims

1. An in vitro method for use in diagnosis of Alzheimer's disease in a human subject which comprises screening for the presence in the genome of said subject of at least one mutation or allelic variant in a cell cycle regulatory gene selected from: CDKN3, p15ink4B, p16ink4A, p19ink4D, p27kip1, p21cip1, p57kip2 and TP53, wherein the presence of a mutation or allelic variant in a cell cycle regulatory gene is taken as an indication of Alzheimer's disease.

2. An in vitro method of determining any genetic basis for Alzheimer's disease in a human subject, which comprises screening the genome of said subject for the presence of mutations or allelic variants in a cell cycle regulatory gene selected from: CDKN3, p15ink4B, p16ink4A, p19ink4D, p27kip1, p21cip1, p57kip2 and TP53.

3.  An in vitro method of screening a human subject for pre-disposition to Alzheimer's disease which comprises screening for the presence in the genome of said subject of at least one mutation or allelic variant in a cell cycle regulatory gene selected from: CDKN3, p15ink4B, p16ink4A, p19ink4D, p27kip1, p21cip1, p57kip2 and TP53.

4.  A method according to any of claims 1-3 which comprises genotyping for one or more of the p21E2 A/B polymorphism, the p57E2A A/B polymorphism, or the p57E2B A/B polymorphism.

**Patentansprüche**

1.  *In vitro*-Verfahren zur Verwendung bei der Diagnose von Alzheimer-Krankheit bei einem menschlichen Individuum, wobei das Verfahren das Screening auf das Vorhandensein zumindest einer Mutation oder Allelvariante in einem regulatorischen Gen des Zellzyklus, das unter: CDKN3, p15ink4B, p16ink4A, p19ink4D, p27kip1, p21cip1, p57kip2 und TP53 ausgewählt ist, in dem Genom des Individuums umfasst, wobei das Vorhandensein einer Mutation oder Allelvariante in einem regulatorischen Gen des Zellzyklus als Anzeichen für die Alzheimer-Krankheit gewertet wird.

2.  *In vitro*-Verfahren zur Bestimmung einer genetischen Basis für die Alzheimer-Krankheit bei einem menschlichen Individuum, wobei das Verfahren das Screening auf das Vorhandensein zumindest einer Mutation oder von Allelvarianten in einem regulatorischen Gen des Zellzyklus, das unter: CDKN3, p15ink4B, p16ink4A, p19ink4D, p27kip1, p21cip1, p57kip2 und TP53 ausgewählt ist, in dem Genom des Individuums umfasst.

3.  *In vitro*-Verfahren zum Screening eines menschlichen Individuums auf eine Prädisposition für Alzheimer-Krankheit, wobei das Verfahren das Screening auf das Vorhandensein zumindest einer Mutation oder Allelvariante in einem regulatorischen Gen des Zellzyklus, das unter: CDKN3, p15ink4B, p16ink4A, p19ink4D, p27kip1, p21cip1, p57kip2 und TP53 ausgewählt ist, in dem Genom des Individuums umfasst.

4.  Verfahren nach einem der Ansprüche 1 bis 3, das eine Genotypisierung bezüglich p21 E2 A/B-Polymorphismus und/oder p57E2A A/B-Polymorphismus und/oder p57E2B A/B-Polymor-phismus umfasst.

**Revendications**

1.  Procédé in vitro pour une utilisation dans le diagnostic de la maladie d'Alzheimer chez un sujet humain, qui comprend le criblage pour déterminer la présence dans le génome dudit sujet d'au moins une mutation ou un variant allélique dans un gène de régulation du cycle cellulaire choisi entre : CDKN3, p15ink4B, p16ink4A, p19ink4D, p27kip1, p21cip1, p57kip2 et TP53, où la présence d'une mutation ou d'un variant allélique dans un gène de régulation du cycle cellulaire est considérée comme une indication de la maladie d'Alzheimer.

2.  Procédé in vitro de détermination de toute base génétique de la maladie d'Alzheimer chez un sujet humain, qui comprend le criblage du génome dudit sujet pour déterminer la présence de mutations ou de variants alléliques dans un gène de régulation du cycle cellulaire choisi entre : CDKN3, p15ink4B, p16ink4A, p19ink4D, p27kip1, p21cip1, p57kip2 et TP53.

3.  Procédé in vitro de criblage chez un sujet humain pour déterminer la prédisposition à la maladie d'Alzheimer qui comprend le criblage pour déterminer la présence dans le génome dudit sujet d'au moins une mutation ou un variant allélique dans un gène de régulation du cycle cellulaire choisi entre : CDKN3, p15ink4B, p16ink4A, p19ink4D, p27kip1, p21cip1, p57kip2 et TP53.

4.  Procédé selon l'une des revendications 1 à 3, qui comprend le génotypage d'un ou de plusieurs parmi le polymorphisme p21E2 A/B, le polymorphisme p57E2A A/B et le polymorphisme p57E2B A/B.

Figure 1. Flow cytometer readouts from a control subject, preAD subject and AD patient.

| Diagnosis | Control Experiment | Rapamycin treatment |
|---|---|---|
| Control | | |
| PreAD | | |
| AD | | |

Figure 2. Relative and age-corrected relative lenghtening of the G1 phase under the influence of Rapamicin.

Figure 3. Effects of 24 hours rapamicin treatment on cell survival.

Figure 4. Effects of doxorubicine treatment on cell survival

Figure 5. Effects of H₂O₂ treatment on cell survival.

Figure 6. PCR-SSCP analysis for p21 exon2, p57 exon 2 fragments 2A and 2B

**p21 exon 2    p57 exon 2A   p57 exon2B**

A B    A B    A B

Figure 7. Relationship between p21 variants A and B and cyclin expression in the brain.

Figure 8. Relationship between p57 exon 2A variants A and B and cyclin expression in the brain in patients with normal p21 (allele B).

Figure 9. The prevalence of somatic mutations in relation to AD progression and cell cycle proteins in the brain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0079000 A
- WO 9738133 A

**Non-patent literature cited in the description**

- **MCKHANN, G. et al.** *Neurology,* 1984, vol. 34, 939-944
- **MOSMANN, T.** *In J. Immunol. Methods,* 1983, vol. 65, 55-63
- **SCHAFER ; HAWKINS.** *Nature Biotechnology,* 1998, vol. 16, 33-39
- **ARAGA S ; KAGIMOTO H ; FUNAMOTO K ; TAKAHASHI K.** *Jpn J Med,* 1990, vol. 29, 572-5
- **ARENDT T ; HOLZER M ; GARTNER U.** *J Neural Transm,* 1998, vol. 105, 949-60
- **ARENDT T ; RODEL L ; GARTNER U ; HOLZER M.** *Neuroreport,* 1996, vol. 7, 3047-9
- **BURKE WJ ; MCLAUGHLIN JR ; CHUNG HD ; GILLESPIE KN ; GROSSBERG GT ; LUQUE FA ; ZIMMERMAN J.** *Alzheimer Dis Assoc Disord,* 1994, vol. 8, 22-8
- **DARZYNKIEWICZ Z.** The cell cycle. Oxford University Press, 1993, 43-68
- **DAVIES KJ.** *IUBMB Life,* 1999, vol. 48, 41-7
- **DRABKIN HA ; ERICKSON P.** *Prog Clin Biol Res,* 1995, vol. 393, 169-76
- **ECKERT A ; HARTMANN H ; FORSTL H ; MULLER WE.** *Life Sci,* 1994, vol. 55, 2019-29
- **FISCHMAN HK ; REISBERG B ; ALBU P ; FERRIS SH ; RAINER JD.** *Biol Psychiatry,* 1984, vol. 19, 319-27
- **FONG CT ; BRODEUR GM.** *Cancer Genet Cytogenet,* 1987, vol. 28, 55-76
- **MECOCCI P ; POLIDORI MC ; INGEGNI T ; CHERUBINI A ; CHIONNE F ; CECCHETTI R ; SENIN U.** *Neurology,* 1998, vol. 51, 1014-1017
- **MELARAGNO MI ; SMITH MDA ; KORMANN BORTOLOTTO MH ; TONIOLO NETO JT.** *Gerontology,* 1991, vol. 37, 293-8
- **NAGY Z ; ESIRI MM ; HINDLEY NJ ; JOACHIM C ; MORRIS JH ; KING EM-F ; MCDONALD B ; LITCHFIELD S ; BARNETSON L ; JOBST KA.** *Dementia,* 1998, vol. 9, 219-226
- **NAGY Z ; ESIRI MM ; SMITH AD.** *Neuroscience,* 1998, vol. 84, 731-739
- **PAYAO SL ; SMITH MD ; BERTOLUCCI PH.** *Gerontology,* 1998, vol. 44, 267-71
- **SHERR CJ.** *Stem Cells,* 1994, vol. 12, 47-55
- **TATEBAYASHI Y ; TAKEDA M ; KASHIWAGI Y ; OKOCHI M ; KURUMADANI T ; SEKIYAMA A ; KANAYAMA G ; HARIGUCHI S ; NISHIMURA T.** *Dementia,* 1995, vol. 6, 9-16
- **TRIEB K ; RANSMAYR G ; SGONC R ; LASSMANN H ; GRUBECK LOEBENSTEIN B.** *Neurobiol Aging,* 1996, vol. 17, 541-7
- **WAGNER EF ; HLEB M ; HANNA N ; SHARMA S.** *J Immunol,* 1998, vol. 161, 1123-31
- **NAGY Z.** *Neurobiol Aging,* 2000, vol. 21, 761-9
- **MCSHEA A ; HARRIS PLR ; WEBSTER KR ; WAHL AF ; SMITH MA.** *Am J Pathol,* 1997, vol. 150, 1933-9

SEQUENCE LISTING

<110> ISIS INNOVATION LIMITED

<120> DIAGNOSTIC SCREENS FOR ALZHEIMER'S DISEASE

<130> SCB/56769/001

<160> 16

<170> PatentIn version 3.1

<210> 1
<211> 28
<212> DNA
<213> Artificial sequence

<220>
<223> PCR-SSCP primer for p21

<400> 1
cgggatccgg cgccatgtca gaaccggc          28

<210> 2

<211> 20
<212> DNA
<213> Artificial sequences

<220>
<223> PCR-SSCP primer for p21

<400> 2
ccagacaggt cagcccttgg        20

<210> 3
<211> 18
<212> DNA
<213> Artificial sequence

<220>
<223> PCR-SSCP primer for p57

<400> 3
ggccatgtcc gacgcgtc        18

<210> 4
<211> 20
<212> DNA
<213> Artificial sequence

<220>
<223> PCR-SSCP primer for p57

<400> 4
aggcggcagc gccccacctg        20

<210> 5
<211> 23
<212> DNA
<213> Artificial sequence

<220>
<223> PCR-SSCP primer for p57

<400> 5
attacgactt ccagcaggac atg        23

<210> 6
<211> 21
<212> DNA
<213> Artificial sequence

<220>
<223> PCR-SSCP primer for p57

<400> 6
ctggagccag gaccgggact g        21

<210> 7
<211> 10
<212> DNA
<213> Artificial sequence

<220>
<223> RAPD primer

<400> 7
ccggctacgg          10

<210> 8
<211> 10
<212> DNA
<213> Artificial sequence

<220>
<223> RAPD primer

<400> 8
caggcccttc          10

<210> 9
<211> 10
<212> DNA
<213> Artificial sequence

<220>
<223> RAPD primer

<400> 9
tacggacacg          10

<210> 10
<211> 10
<212> DNA
<213> Artificial sequence

<220>
<223> RAPD primer

<400> 10
agcttcaggg          10

<210> 11
<211> 10
<212> DNA
<213> Artificial sequence

<220>
<223> RAPD primer

<400> 11
aggcattccc          10

<210> 12
<211> 10
<212> DNA
<213> Artificial sequence

<220>
<223> RAPD primer

<400> 12
ggtctgaacc        10

<210> 13
<211> 10
<212> DNA
<213> Artificial sequence

<220>
<223> RAPD primer

<400> 13
taggctcacg        10

<210> 14
<211> 10
<212> DNA
<213> Artificial sequence

<220>
<223> RAPD primer

<400> 14
acggtacact        10

<210> 15
<211> 10
<212> DNA
<213> Artificial sequence

<220>
<223> RAPD primer

<400> 15
gtcctcaacg        10

<210> 16
<211> 10
<212> DNA
<213> Artificial sequence

<220>
<223> RAPD primer

<400> 16
caatgcgtct        10